(19) Europäisches Patentamt
European Patent Office
Office européen des brevets

(11) Publication number: **0 312 657**
**A1**

(12) **EUROPEAN PATENT APPLICATION**

(21) Application number: **87202040.9**

(51) Int. Cl.4: **G03C 7/32**

(22) Date of filing: **23.10.87**

(43) Date of publication of application:
**26.04.89 Bulletin 89/17**

(84) Designated Contracting States:
**AT BE CH DE ES FR GB GR IT LI LU NL SE**

(71) Applicant: **AGFA-GEVAERT naamloze vennootschap**
**Septestraat 27**
**B-2510 Mortsel(BE)**

(72) Inventor: **Janssens,Wilhelmus**
**De Egdstraat 11**
**B-3240 Aarschot(BE)**
Inventor: **Van de Sande,Christian Charles**
**Tulnlaan 95**
**B-9180 Belsele(BE)**
Inventor: **Monbaliu,Marcel Jacob**
**Neuris 9**
**B-2510 Mortsel(BE)**

(54) **Colour photographic elements incorporating alkali-labile couplers.**

(57) Colour photographic element comprising a support and coated thereon at least one alkali-permeable light-sensitive silver halide emulsion layer incorporating in said emulsion layer or in an adjacent layer in water-permeable relationship therewith a non-diffusing colour coupler capable of reacting in alkaline medium with the oxidation product of a p-phenylene diamine colour developing agent to form a cyan, magenta, or yellow dye, and said coupler being an alkali-labile coupler, whose capability to enter into a coupling reaction with oxidized p-phenylene diamine colour developing agent in said alkaline medium is gradually lost as a result of an alkali-induced reaction of said coupler. The invention also provides a method of avoiding lack of definition in dye images produced by chromogenic development including a coupling reaction in alkaline medium between oxidized p-phenylene diamine developing agent and a said alkali-labile coupler.

EP 0 312 657 A1

# COLOR PHOTOGRAPHIC ELEMENTS INCORPORATING ALKALI-LABILE COUPLERS

The present invention relates to colour photographic elements incorporating alkali-labile colour couplers, by means of which by chromogenic development dye images having improved sharpness are produced.

It is generally known that by the use of DIR-couplers (Development-inhibitor-releasing-couplers) or DIR-compounds (Development-inhibitor-releasing-compounds) in colour photographic silver halide elements a number of photographic characteristics that determine the quality of the colour image produced therein can be influenced advantageously. It is indeed known to carry out the chromogenous development of colour photographic silver halide elements in the presence of compounds that during development imagewise release diffusible compounds capable of inhibiting the development of silver halide. Such compounds are called DIR-couplers, when they are of the type that enters into reaction with oxidized colour developing agent to form a coloured dye whilst releasing a development-inhibiting fragment or they are called DIR-compounds when they are of the type that releases a development-inhibiting fragment without simultaneously forming a coloured dye. DIR-couplers are known from e.g. US-A 3,148,062, US-A 3,227,554, US-A 3,615,506, and US-A 3,617,291. With respect to DIR-compounds that form substantially colourless coupling products, reference can be made to e.g. US-A 3,632,345, DE-A 2,359,295, and DE-A 2,540,959.

By the use of DIR-couplers and DIR-compounds a reduced gradation, a reduced grain, improved sharpness by the so-called edge effect, improved colour purity and brillance can be obtained. Reference can be made with respect to these effects to "Development-Inhibitor-Releasing (DIR) couplers in Color Photography" by C.R. Barr, J.R. Thirtle, and P.W. Vittum, Photographic Science and Engineering 13, 74-80 and 214-219 (1969). The released development-inhibitors usually are heterocyclic mercapto compounds or benzotriazole derivatives.

In spite of the improvements accomplished so far by the use of DIR-couplers and DIR-compounds in colour photographic silver halide elements, the image produced therein is sometimes still insufficiently sharp. Insufficient sharpness is particularly disturbing in the case of colour photographic elements, in which the image production should occur with the highest possible definition. Especially in colour negative motion picture elements a high definition is of utmost importance.

It has been established that lack of definition in dye images produced by chromogenic development i.e. by reaction between oxidized developing agent and coupler(s) can be due to the following phenomenon. Inasmuch as the amount of coupler present in a light-sensitive silver halide emulsion layer of a colour photographic element or in an adjacent layer in water-permeable relationship therewith is by far exceeded stoichiometrically by the amount of silver halide present in the emulsion layer, the amount of developing agent that is oxidized at the exposed areas during chromogenic development is consequently also largely in excess of that of the coupler present there. During the initial stage of the chromogenic development the colour developing agent that is oxidized to form a quinonediimine, can enter into reaction with coupler in the immediate vicinity and thus form a sharp dye image. However, as development of the exposed silver halide goes on, a relatively high amount of quinonediimine is still present at the exposed areas whereas the amount of coupler available at these same areas, especially at the areas exposed more intensively, reaches an end. As a consequence the excess quinonediimine tends to diffuse laterally to find coupler, where it can still find it. This means that at the edges of the image areas quinonediimine can diffuse into adjacent unexposed areas and form a dye with coupler still available there. It is self-evident that the formation of dye in these areas results in the formation of an unsharp dye image.

It is therefore an object of the present invention to provide colour photographic elements incorporating colour couplers, by means of which dye images can be produced by chromogenic development, these dye images having an improved sharpness.

According to the present invention a colour photographic element has been found, which comprises a support and coated thereon at least one alkali-permeable light-sensitive silver halide emulsion layer incorporating in said emulsion layer or in an adjacent layer in water-permeable relationship therewith at least one non-diffusing colour coupler, preferably at least one non-diffusing colour coupler chosen from the group consisting of cyan-forming phenol and naphthol couplers, magenta-forming 2-pyrazolin-5-one couplers, and yellow-forming acylacetamide couplers, said at least one non-diffusing colour coupler being capable of reacting in alkaline medium with the oxidation product of a p-phenylene diamine colour developing agent to form a cyan, magenta, or yellow dye, characterized in that said non-diffusing colour coupler is an alkali-labile coupler, whose capability to enter into a coupling reaction with oxidized p-phenylene diamine colour developing agent in said alkaline medium is gradually lost as a result of an alkali-induced reaction e.g. a cyclization reaction or a release reaction of said coupler.

By the expression "alkali-labile" as used herein is meant that couplers having the property of being alkali-labile, gradually loose their capacity of entering into reaction with oxidized colour developing agent when placed in an alkaline medium and that this loss in reactivity is the more important, the higher the pH-value of said alkaline medium. By the expression "alkali-stable" as used herein is meant the opposite, in other words alkali-stable couplers retain their coupling capacity in alkaline processing baths.

Alkali-labile non-diffusing couplers that can be used in accordance with the present invention belong to one of the following classes:

- alkali-labile cyan-forming phenol and naphthol couplers carrying at the 2-position a $-CH(CH_3)-SO_2-R$ substituent, wherein R represents an aryl group or a substituted aryl group,
- alkali-labile cyan-forming phenol and naphthol couplers carrying at the 2-position a -NHCO-A-X substituent, wherein A is a polyvalent hetero-atom e.g. -O- and -S- and X is an aromatic ring system e.g. phenyl and naphthyl or is a heterocyclic ring system,
- alkali-labile magenta-forming 2-pyrazolin-5-one couplers, which on the 1-position carry a phenyl nucleus having in ortho-position a -CO-A-X substituent, wherein A and X have a significance as defined above,
- alkali-labile yellow-forming benzoylacetamide couplers, which in ortho-position on the benzene ring of the benzoyl group carry a -NHCO-A-X substituent, wherein A and X have a significance as defined above, and
- alkali-labile yellow-forming acylacetanilide couplers, which in ortho-position on the benzene ring of the anilide part carry a -NHCO-A-X substituent, wherein A and X have a significance as defined above,

wherein all of said couplers may carry further substituent(s) as customarily used in the art of coupler chemistry e.g. coupling-off groups.

Preferentially used alkali-labile non-diffusing couplers, which can be prepared very conveniently and are very interesting from an economical standpoint, are those of the following classes:

- alkali-labile cyan-forming couplers corresponding to the general formula I:

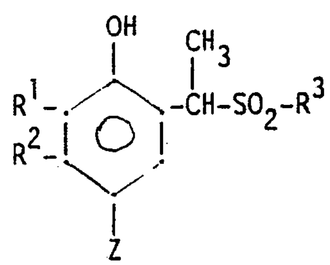

(I)

wherein:

each of $R^1$ and $R^2$, which may be the same or different, is a substituent chosen from the group consisting of hydrogen, a halogen atom e.g. chloro, a $C_1$-$C_{18}$ alkyl group e.g. methyl and pentadecyl, a substituted $C_1$-$C_{18}$ alkyl group, a $C_1$-$C_{18}$ alkoxy group, a substituted $C_1$-$C_{18}$ alkoxy group, an acylamido group, a substituted acylamido group, an ureido group, and a substituted ureido group, or $R^1$ and $R^2$ together represent the atoms necessary to complete a fused on carbocyclic ring e.g. a benzene nucleus or a substituted benzene nucleus,

$R^3$ represents an aryl group e.g. phenyl or a substituted aryl group e.g. tolyl,

Z is hydrogen in the case of a 4-equivalent coupler or a so-called coupling off group, which splits off upon colour development, thus conferring to the colour coupler a 2-equivalent character e.g. a halogen atom such as chlorine, an acyloxy group, an alkoxy group, a substituted alkoxy group, an aryloxy group, a substituted aryloxy group, a heterocycloxy group, a substituted heterocycloxy group, an alkylthio group, a substituted alkylthio group, an arylthio group e.g. phenylthio, a substituted arylthio group e.g. carboxyphenylthio, an alkylsulphonyl group, a substituted alkylsulphonyl group, an arylsulphonyl group, a substituted arylsulphonyl group, an alkylsulphinyl group, a substituted alkylsulphinyl group, an arylsulphinyl group, a substituted arylsulphinyl group, an alkyl- or aryl-substituted carbonylmethoxy group, an alkoxy- or aryloxy-substituted carbonylmethoxy group, a heterocyclyl-thio group such as a tetrazolylthio group, or a phenylazo group,

- alkali-labile cyan-forming couplers corresponding to the general formula II:

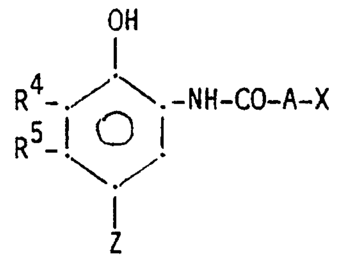

(II)

wherein:

Z has the significance as defined for Z in general formula I,

$R^4$ is a substituent chosen from the group consisting of hydrogen, chloro, an alkyl group, a substituted alkyl group, an alkenyl group, and a substituted alkenyl group,

$R^5$ is a substituent chosen from the group consisting of a $C_5$-$C_{18}$ alkyl group, a substituted $C_5$-$C_{18}$ alkyl group, and a $R^6$-CONH- group e.g. 2-(2,4-di-t.pentylphenoxy)-hexanamido, $R^6$ standing for a $C_5$-$C_{18}$ alkyl group or a substituted $C_5$-$C_{18}$ alkyl group, or

$R^4$ and $R^5$ together represent the atoms necessary to complete a heterocyclic nucleus e.g. -NH-CO-O-, -NH-CO-S-, -NH-CO-$NR^7$-, -NH-CO-C($R^8R^9$)-, -NH-CO-C($R^8R^9$)-$NR^7$-, -NH-CO-C($R^8R^9$)-O-, -NH-CO-C-($R^8R^9$)-S-, wherein $R^7$ is a substituent chosen from the group consisting of hydrogen, an alkyl group, a substituted alkyl group, an aryl group, and a substituted aryl group, and wherein $R^8$ and $R^9$ can be identical or different and are chosen from the group consisting of a halogen atom, cyano, an alkyl group, a substituted alkyl group, an aryl group, a substituted aryl group, an alkoxy group, a substituted alkoxy group, an alkylcarbonyl group, a substituted alkylcarbonyl group, an arylcarbonyl group, a substituted arylcarbonyl group, an alkoxycarbonyl group, a substituted alkoxycarbonyl group, a carbamoyl group, a substituted carbamoyl group, an acylamido group, and a substituted acylamido group, or $R^8$ and $R^9$ together form an oxo group,

A is a polyvalent hetero-atom e.g. -O- and -S-,

X is an aromatic ring system e.g. phenyl and naphthyl or is a heterocyclic ring system,

wherein the moiety -A-X is cleavable or releasable gradually from the coupler molecule during prolonged stay in an alkaline medium,

- alkali-labile magenta-forming couplers corresponding to the general formula III:

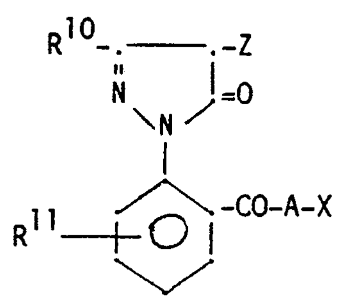

(III)

wherein:

Z has the significance as defined for Z in general formula I,

$R^{10}$ represents a substituent chosen from the group consisting of an alkyl group, a substituted alkyl group, an alkenyl group, a substituted alkenyl group, an aryl group, a substituted aryl group, an alkoxycarbonyl group, a substituted alkoxycarbonyl group, an aryloxycarbonyl group, a substituted aryloxycarbonyl group, an alkoxy group, a substituted alkoxy group, an aryloxy group, a substituted aryloxy group, an alkylthio group, a substituted alkylthio group, an arylthio group, a substituted arylthio group, amino, a substituted amino group, a cycloalkylamino group, a substituted cycloalkylamino group, amido, a substituted amido group, anilino, a substituted anilino group, a N-alkylacylamido group, a substituted N-alkylacylamido group, a N-arylacylamido group, a substituted N-arylacylamido group, a N-alkylureido group, a substituted N-alkylureido group, a N-arylureido group, a substituted N-arylureido group, a N-alkylthioureido group, a substituted N-alkylthioureido group, a carbamoyl group, a substituted carbamoyl group, cyano, a guanidino group, a substituted guanidino group, a heterocyclyl group, a substituted heterocyclyl group, an alkyl-substituted sulphonylamido group, and an aryl-substituted sulphonylamido group,

$R^{11}$ represents a substituent chosen from the group consisting of hydrogen, a halogen atom e.g. 2-chloro and 3-chloro, an alkyl group e.g. 2-methyl and 3-methyl, a substituted alkyl group, an alkoxy group e.g. 2-methoxy and 3-methoxy, a substituted alkoxy group, cyano, an acylamido group, a substituted acylamido group, an alkyl-substituted carbamoyl group, an aryl-substituted carbamoyl group, an alkylsulphonyl group, a substituted alkylsulphonyl group, an arylsulphonyl group, a substituted arylsulphonyl group, an alkylthio group, a substituted alkylthio group, an arylthio group, and a substituted arylthio group,

A has the significance as defined above,

X has the significance as defined above,

wherein the moiety -A-X is cleavable or releasable gradually from the coupler molecule during prolonged stay in an alkaline medium,

- alkali-labile yellow-forming couplers corresponding to the general formula IV:

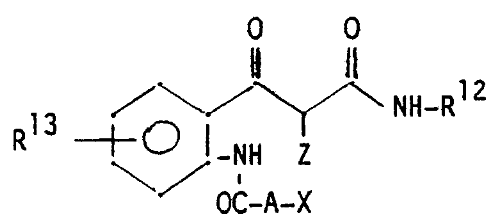

(IV)

wherein:

Z has the significance as defined for Z in general formula I,

$R^{12}$ represents phenyl or phenyl substituted by 1 to 3 substituents, which may be the same or different and are selected from the group consisting of a branched or unbranched $C_1$-$C_{18}$ alkyl group, the alkyl group being substituted or not, an $C_1$-$C_{18}$ alkoxy group e.g. methoxy and n-hexadecyloxy, a substituted $C_1$-$C_{18}$ alkoxy group, a halogen atom e.g. chloro, an acylamido group, a substituted acylamido group e.g. (di)-alkyl-substituted phenoxyacylamido, a carbamoyl group, a substituted carbamoyl group, a sulphamoyl group, a substituted sulphamoyl group e.g. N,N-dialkyl-substituted sulphamoyl, carboxy, nitro, cyano, an alkylsulphonyl group, a substituted alkylsulphonyl group, an arylsulphonyl group, a substituted arylsulphonyl group, an alkoxycarbonyl group, a substituted alkoxycarbonyl group, an aryloxycarbonyl group, a substituted aryloxycarbonyl group, an alkylthio group, a substituted alkylthio group, an arylthio group, and a substituted arylthio group,

$R^{13}$ represents a substituent chosen from the group consisting of hydrogen, a branched or unbranched $C_1$-$C_{18}$ alkyl group, the alkyl group being substituted or not, an $C_1$-$C_{18}$ alkoxy group e.g. methoxy and n-hexadecyloxy, a substituted $C_1$-$C_{18}$ alkoxy group, a halogen atom e.g. chloro, an acylamido group, a substituted acylamido group e.g. (di)alkyl-substituted phenoxyacylamido, a carbamoyl group, a substituted carbamoyl group, a sulphamoyl group, a substituted sulphamoyl group e.g. N,N-dialkyl-substituted sulphamoyl, carboxy, nitro, cyano, an alkylsulphonyl group, a substituted alkylsulphonyl group, an arylsulphonyl group, a substituted arylsulphonyl group, an alkoxycarbonyl group, a substituted alkoxycarbonyl group, an aryloxycarbonyl group, a substituted aryloxycarbonyl group, an alkylthio group, a substituted alkylthio group, an arylthio group, and a substituted arylthio group,

A has the significance as defined above,

X has the significance as defined above,

wherein the moiety -A-X is cleavable or releasable gradually from the coupler molecule during prolonged stay in an alkaline medium,

- alkali-labile yellow-forming couplers corresponding to the general formula V:

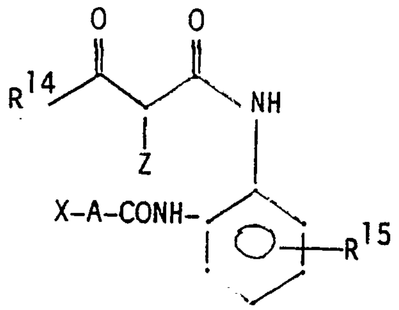

(V)

wherein:

Z has the significance as defined for Z in general formula I,

$R^{14}$ represents a substituent chosen from the group consisting of a branched or unbranched $C_1$-$C_{18}$ alkyl group, wherein in the case of a secondary or tertiary alkyl group the secondary or tertiary carbon atom is preferably linked directly to the vicinal carbonyl group e.g. a tert-butyl group, an alkoxyalkyl group, a substituted alkoxyalkyl group, a cycloalkyl group, a substituted cycloalkyl group, a heterocyclyl group, a substituted heterocyclyl group, an aryl group e.g. phenyl, and an aryl group substituted with a substituent chosen from the group consisting of alkyl, alkoxy e.g. methoxyphenyl and n-hexadecyloxyphenyl, a halogen atom, cyano, an acyl group, a substituted acyl group, an acylamido group, a substituted acylamido group, a carbamoyl group, a substituted carbamoyl group, a sulphamoyl group, a substituted sulphamoyl group, a sulphonamido group, a substituted sulphonamido group, carboxy, an alkylthio group, a substituted alkylthio group, an arylthio group, and a substituted arylthio group,

$R^{15}$ represents a substituent chosen from the group consisting of hydrogen, a branched or unbranched $C_1$-$C_{18}$ alkyl group, the alkyl group being substituted or not, an $C_1$-$C_{18}$ alkoxy group e.g. methoxy and n-hexadecyloxy, a substituted $C_1$-$C_{18}$ alkoxy group, a halogen atom e.g. chloro, an acylamido group, a substituted acylamido group e.g. (di)alkyl-substituted phenoxyacylamido, a carbamoyl group, a substituted carbamoyl group, a sulphamoyl group, a substituted sulphamoyl group e.g. N,N-dialkyl-substituted sulphamoyl, carboxy, nitro, cyano, an alkylsulphonyl group, a substituted alkylsulphonyl group, an arylsulphonyl group, a substituted arylsulphonyl group, an alkoxycarbonyl group, a substituted alkoxycarbonyl group, an aryloxycarbonyl group, a substituted aryloxycarbonyl group, an alkylthio group, a substituted alkylthio group, an arylthio group, and a substituted arylthio group,

A has the significance as defined above,

X has the significance as defined above,

wherein the moiety -A-X is cleavable or releasable gradually from the coupler molecule during prolonged stay in an alkaline medium.

The present invention also provides a method of avoiding lack of definition in dye images produced by chromogenic development including a coupling reaction in alkaline medium between oxidized p-phenylene diamine developing agent and at least one non-diffusing colour coupler chosen from the group consisting of cyan-forming phenol and naphthol couplers, magenta-forming 2-pyrazolin-5-one couplers, and yellow-forming acylacetamide couplers, said lack of definition being caused by the coupling reaction taking place also undesiredly between said coupler and any part of said oxidized p-phenylene diamine developing agent that towards the end of said chromogenic development laterally diffuses at the edges of the image areas, characterized in that towards the end of said chromogenic development said at least one coupler is gradually rendered incapable of taking part in said coupling reaction as a consequence of an alkali-induced inactivation reaction of said coupler, said inactivation reaction being controlled by the alkalinity of said alkaline medium and in proportion to the degree of said alkalinity.

It has been established indeed that by chromogenic development of a colour photographic element according to the present invention comprising an alkali-labile colour coupler, sharp dye images can be produced thanks to the fact that during this chromogenic development the alkali-labile colour coupler contained in said element gradually looses its capacity of coupling with oxidized colour developing agent i.e. quinonediimine. This gradual extinguishment of the coupling capacity is effected proportionally with the stay of said coupler in the alkaline medium of the developer solution and with the pH-value of said medium. Towards the end of the development period the alkali-labile coupler has lost its ability to couple so that any quinonediimine, which at that very stage of development often starts diffusing laterally from the edges of the exposed areas towards the unexposed areas in its search for still available coupler, does not find reactive coupler anymore. The extinguishment of the coupling capacity of the alkali-labile couplers according to the

present invention is thus caused by an inactivation reaction taking place slowly and gradually in alkaline medium.

The coupling reaction that can take place between a quinonediimine and the alkali-labile couplers used in accordance with the present invention before inactivation of the latter and, alternatively, the inactivation reaction can be represented by the following reaction schemes (a) to (e), in which the symbols $R^1$ to $R^{15}$ - (whenever exhibited), Z, A, and X have the significances as defined above, and each of $R^{16}$ and $R^{17}$ (same or different) represent a lower alkyl group e.g. methyl and ethyl.

-alkali-labile cyan-forming coupler corresponding to general formula I:

a) coupling reaction

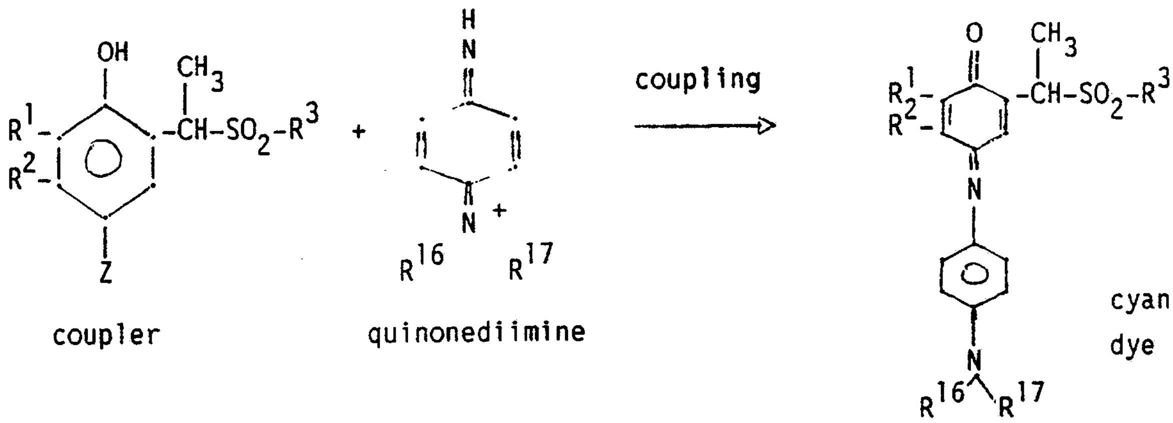


b) inactivation reaction

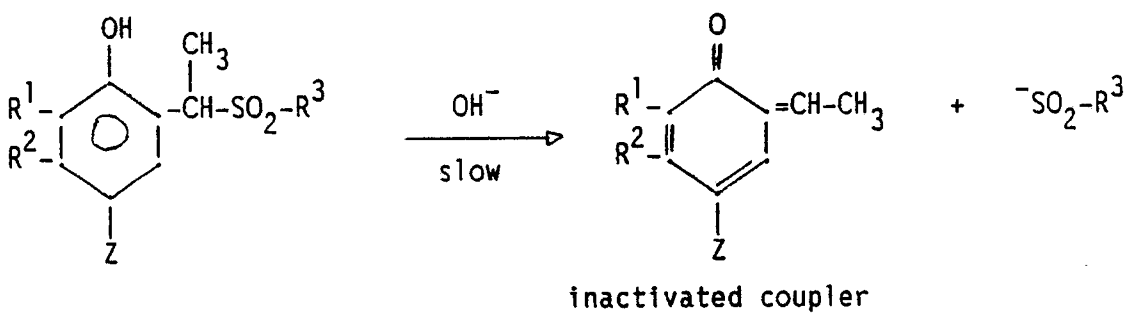


-alkali-labile cyan-forming coupler corresponding to general formula II:

a) coupling reaction

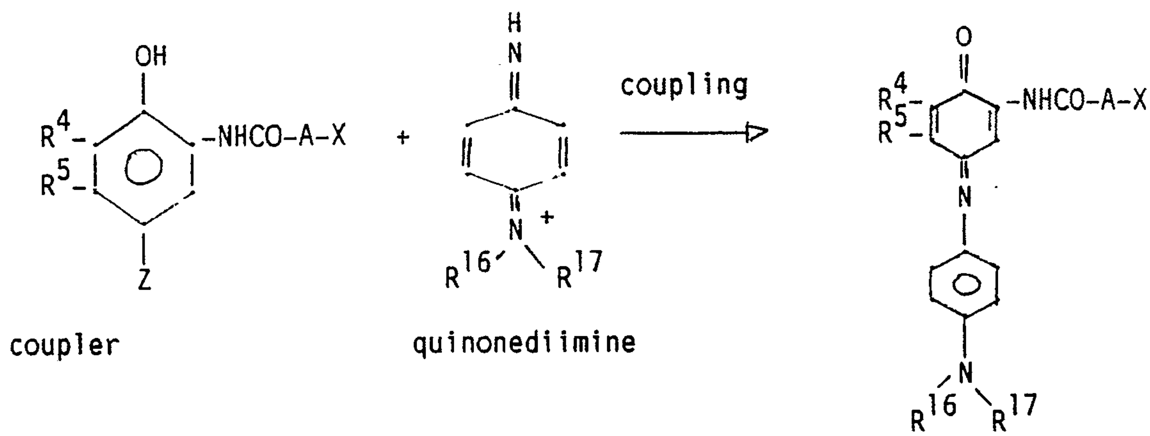


coupler quinonediimine cyan dye

b) inactivation reaction

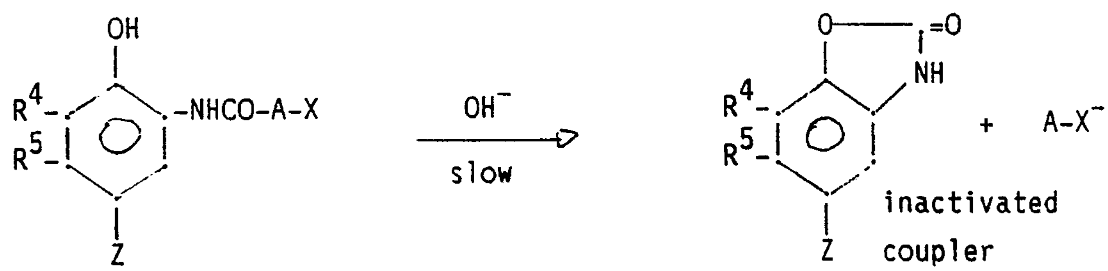


inactivated coupler

-alkali-labile magenta-forming couplers corresponding to the general formula III:

a) coupling reaction

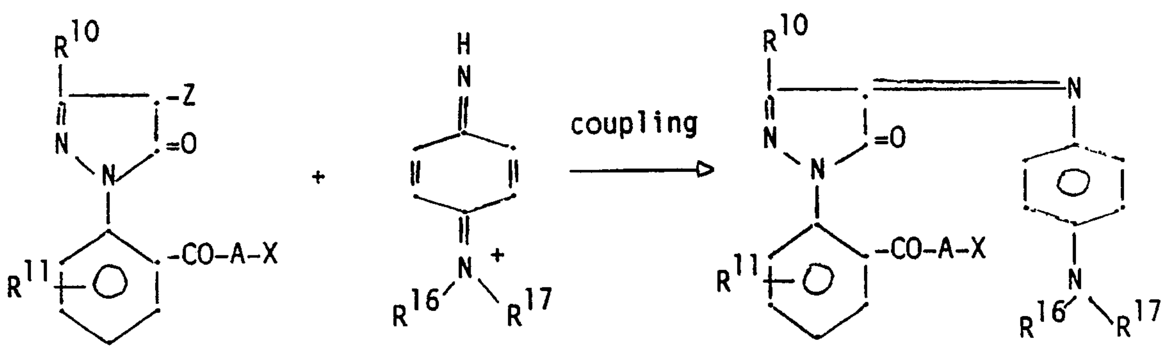


coupler quinonediimine magenta dye

b) inactivation reaction

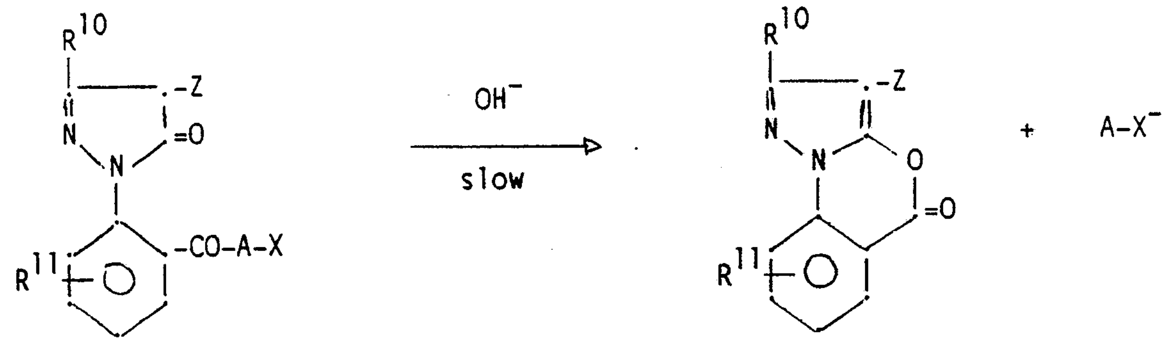


inactivated coupler

-alkali-labile yellow-forming couplers corresponding to the general formula IV:

a) coupling reaction

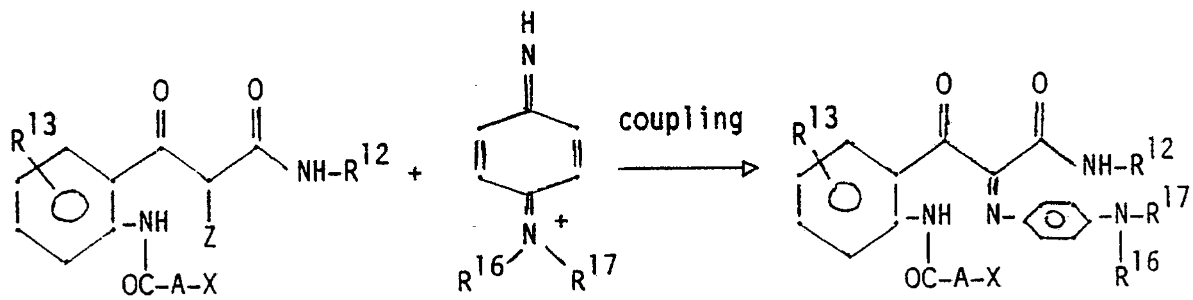


coupler quinonediimine yellow dye

b) inactivation reaction

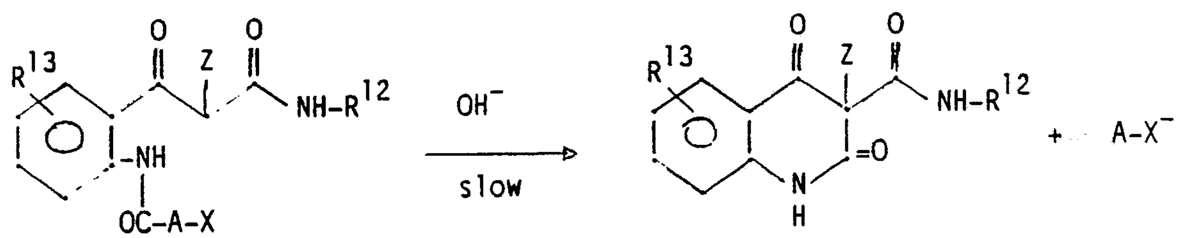


inactivated coupler

-alkali-labile yellow-forming couplers corresponding to the general formula V:

a) coupling reaction

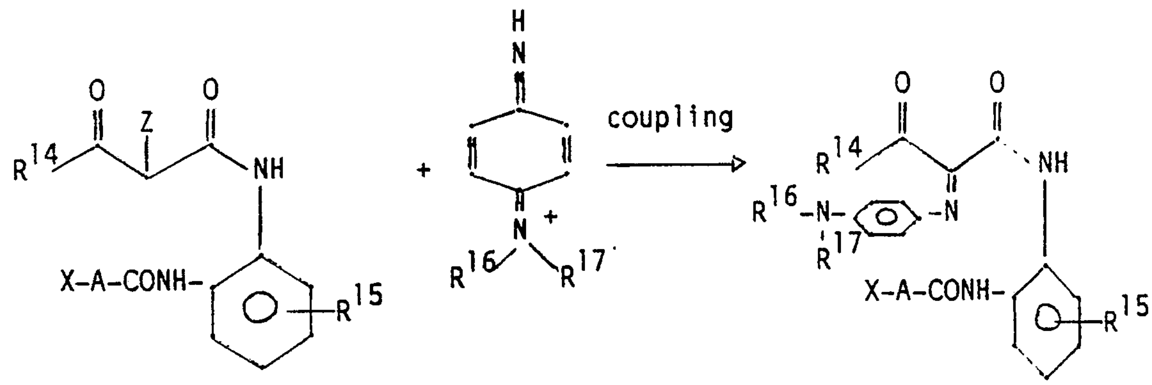


coupler quinonediimine yellow dye

b) inactivation reaction

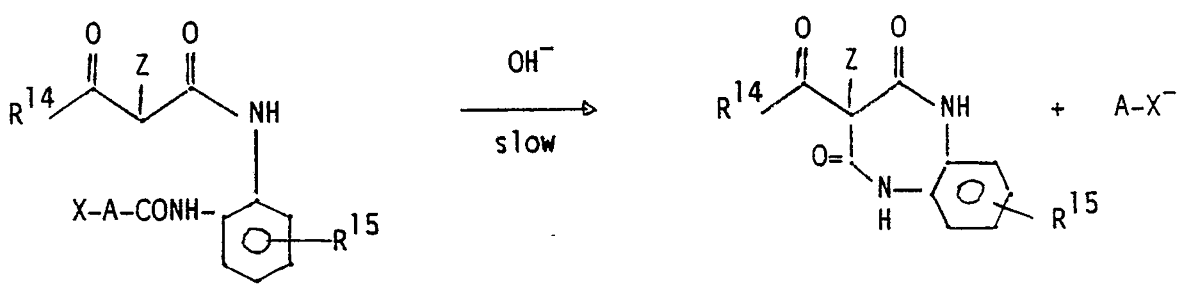


coupler inactivated coupler

In accordance with the present invention all couplers present at the exposed areas as well as at the non-exposed areas are converted during the photographic processing into non-reactive products. As a result, the stability to light, heat, and moisture of the dye images produced in accordance with the present invention is found to be very good.

Representative examples of alkali-labile dye-forming couplers corresponding to the above general formulae I to V are given in the folowing Table 1. It is, however, to be understood that the invention is not limited to the alkali-labile dye-forming couplers specifically identified therein. $R^n$ stands for one of the symbols $R^1$ to $R^{15}$ as specified in Table 1.

TABLE 1

| Coupler | according general formula | Z | A | X | $R^n$ |
|---|---|---|---|---|---|
| 1 | II | H | O | phenyl | $R^5$=2-(2,4-di-t.pentylphenoxy)-hexanamido |
| 2 | II | H | S | phenyl | $R^5$=2-(2,4-di-t.pentylphenoxy)-hexanamido |
| 3 | II | Cl | O | naphthyl | $R^5$=2-(2,4-di-t.pentylphenoxy)-hexanamido |
| 4 | I | H | | | $R^1$=H; $R^2$=pentadecyl; $R^3$=phenyl |
| 5 | III | H | O | phenyl | $R^{10}$=pentadecyl; $R^{11}$=H |
| 6 | IV | H | O | phenyl | $R^{12}$=4-pentadecyloxyphenyl; $R^{13}$=H |
| 7 | V | H | O | phenyl | $R^{14}$=4-pentadecyloxyphenyl; $R^{15}$=H |

The alkali-labile dye-forming couplers corresponding to the above general formulae I to V can be prepared very conveniently and economically as illustrated by the following preparation examples.

The synthesis of other couplers not specifically identified herein but corresponding to the above general formulae I to V can be derived from the preparation examples given hereinafter and will not cause difficulties to those skilled in the art of preparative organic chemistry.

PREPARATION 1: Alkali-labile Coupler 1

An amount of 45.4 g (0.1 mol) of 5-[2-(2,4-di-tert-pentylphenoxy)- hexanamido]-2-aminophenol and 14 g (0.166 mol) of sodium hydrogen carbonate is added to 300 ml of ethyl acetate. The resulting suspension is cooled down to 0 °C and 15.7 g (0.1 mol) of phenyl chloroformate is added dropwise thereto. The reaction mixture is left standing for 2 h at 0 °C and then poured out in 200 ml of water. The ethyl acetate layer is separated, rinsed with a saturated aqueous sodium chloride solution, and dried over magnesium sulphate. The ethyl acetate is removed by evaporation and the residue is recrystallized twice from acetonitrile.
Yield: 47 g (83%). Melting range: 92-97 °C.

PREPARATION 2: Alkali-labile Coupler 4

a) 2-acetyl-5-pentadecyl-phenol

An amount of 152 g (0.5 mol) of m-pentadecylphenol is added to a suspension of 33.5 g (0.25 mol) of anhydrous aluminium chloride in 400 ml of methylene chloride 10-15 °C. With cooling 39.3 g (0.5 mol) of acetyl chloride is added thereto. The reaction mixture is stirred for 1 h at boiling temperature. The solvent is removed by evaporation. Next, 33.5 g (0.25 mol) of anhydrous aluminium chloride is added and the mixture is heated for 1 h at 110 °C. The reaction mixture is poured out in hydrochloric acid. The precipitate formed is separarted, rinsed until neutral, and dried. The residue is recrystallized from methanol.
Yield: 18 g of 2-acetyl-5-pentadecyl-phenol melting at 50 °C.

b) 2-[2-hydroxyethyl]-5-pentadecyl-phenol

An amount of 13.84 g (0.04 mol) of 2-acetyl-5-pentadecyl-phenol and of 80 ml of 2-methoxypropanol is placed in a reaction flask. To the resulting mixture a solution of 1.7 g of $NaBH_4$ (0.44 mol) in 4 ml of water is added dropwise. The temperature rises from 22 to 42 °C. After 30 min a precipitate has formed. The

reaction mixture is poured out in water and neutralized with acetic acid. The precipitate is filtered with suction and dried.
Yield: 12.8 g (92%) of 2-[2-hydroxyethyl]-5-pentadecyl-phenol melting at 62° C.

c) Coupler 4

A mixture of 11.48 g (0.033 mol) of 2-[2-hydroxyethyl]-5-pentadecyl- phenol and 5.42 g (0.033 mol) of the sodium salt of benzenesulfinic acid in 66 ml of acetic acid is heated for 2 h to 70° C. The reaction mixture is poured out in water and the resulting precipitate is filtered, dried, and recrystallized from ethyl acetate.
Yield: 8.5 g of Coupler 4 melting at 101.5° C.

The alkali-labile dye-forming couplers corresponding to the above general formulae I to V are believed to be new compounds and the present invention consequently also includes such compounds per se.

The alkali-labile dye-forming couplers for use in accordance with the present invention are of the non-diffusing type and therefore may comprise in their molecule an organic group sufficiently large to prevent the coupler from wandering from the colloid layer, in which it has been incorporated, to another colloid layer.

In preparing the colour photographic element according to the present invention the alkali-labile non-diffusing dye-forming couplers corresponding to the above general formulae I to V can be incorporated into the coating composition of the silver halide emulsion layers or into other colloid layers in water-permeable relationship therewith according to any technique known by those skilled in the art for incorporating photographic ingredients, more particularly colour couplers, into colloid compositions.

The alkali-labile dye-forming couplers can be dispersed, if needed in the presence of a wetting or dispersing agent, in a hydrophilic composition constituting or forming part of the binding agent of the colloid layer. Very suitable wetting agents that can be used to disperse the alkali-labile dye-forming couplers are the fluorine-containing surface-active agents e.g. those described in EP-A 0,015,592. For more details about particularly suitable techniques that can be employed for incorporating the alkali-labile dye-forming couplers used in accordance with the invention into a hydrophilic colloid layer of a photographic element there can be referred to GB-A 791,219 - 1,098,594 - 1,099,414 - 1,099,415 - 1,099,416 - 1,099,417 - 1,199,570 - 1,218,190 - 1,297,947, to US-A 2,269,158 - 2,284,887 - 2,304,939 - 2,304,940 - 2,322,027, to FR-A 1,555,663, and to BE-A 722,026.

The alkali-labile non-diffusing dye-forming couplers for use in accordance with the present invention are preferably incorporated into a hydrophilic colloid medium from a solution in at least one high-boiling sparingly water-miscible solvent e.g. di-n-butyl phthalate and tricresyl phosphate or in a low-boiling sparingly water-miscible solvent e.g. ethyl acetate, methylene chloride, and chloroform, or in mixtures thereof. Very fine dispersions of non-diffusing dye-forming couplers in hydrophilic colloid media can be obtained by means of these solvents. For this purpose these solutions are dispersed in extremely fine droplets, preferably in the presence of a wetting or dispersing agent, into the hydrophilic colloid medium, the low-boiling sparingly water-miscible solvent being removed afterwards by evaporation.

The alkali-labile non-diffusing dye-forming couplers for use in accordance with the present invention can, of course, also be incorporated in other ways into hydrophilic colloid media. For instance, when the compound can be liquefied by slight heating (in case it has a low melting point) or when it is liquid at room temperature, the liquid can be dispersed as such into the hydrophilic colloid media.

The hydrophilic colloid media, into which the alkali-labile non-diffusing dye-forming couplers for use in accordance with the present invention can be dispersed, need not necessarily be coating compositions for hydrophilic colloid layers. Especially in the case of photographic emulsion components that are to be incorporated into light-sensitive silver halide emulsions, it may be advantageous to first disperse these components into non-light-sensitive hydrophilic colloid compositions or into water, and subsequently to admix the resulting dispersions in their turn with the colloid coating composition such as a silver halide emulsion, from which the hydrophilic colloid layer is to be coated. The hydrophilic colloid media, into which the alkali-labile non-diffusing dye-forming couplers for use in accordance with the present invention can be incorporated, usually comprise gelatin as hydrophilic colloid. However, other water-soluble colloid substances or mixtures thereof can be used too e.g. colloidal albumin, zein, casein, a cellulose derivative such as carboxymethylcellulose, or a synthetic hydrophilic colloid such as polyvinyl alcohol, and poly-N-vinylpyrrolidone.

For more details about suitable dispersing techniques that can be employed for incorporating the alkali-labile non-diffusing dye-forming couplers into a hydrophilic colloid layer of a photographic element,

reference can be made to e.g. US-A 2,269,158 - 2,284,887 - 2,304,939 - 2,304,940, and 2,322,027; GB-A 791,219; FR-A 1,555,663; DE-A 1,127,714, and to BE-A 747,589.

Another technique for incorporating the alkali-labile dye-forming couplers is via polymeric latices as described in DE-A 2,541,230 and 2,541,274 and in Research Disclosure N° 18,815 of December 1979, p.703-8.

The alkali-labile dye-forming couplers for use in accordance with the present invention can be employed in conjunction with various kinds of photographic emulsions e.g. a spectrally sensitized silver halide emulsion, a non-spectrally sensitized silver halide emulsion, a silver halide emulsion of use in diffusion transfer reversal processes, etc. The alkali-labile dye-forming couplers are preferably used in conjunction with photographic emulsions, by means of which dye images having a high definition, have to be produced. Photographic elements that are partucularly apt to work with couplers according to the present invention are the colour negative motion picture elements.

Various silver salts can be used as the light-sensitive salt. For instance silver bromide, silver iodide, silver chloride or mixed silver halides such as silver chlorobromide, silver bromoiodide, and silver chlorobromoiodide can be employed. The couplers can be used in emulsions of the mixed packet type as described in US-A 2,698,794 or emulsions of the mixed grain type as described in US-A 2,592,243. The dye-forming couplers can be used with emulsions, in which latent images are formed predominantly at the surface of the silver halide crystal, or with emulsions, in which latent images are formed predominantly inside the silver halide crystal. The dye-forming couplers can also be used with emulsions of various types such as those described in e.g. US-A 4,425,426 - 4,433,048 - 4,434,226 - 4,435,501 - 4,439,520 - 4,444,877 - 4,514,491 - and EP-A 160,469 - 165,576 - 201,027.

The hydrophilic colloid used as the vehicle for the silver halide can be e.g. gelatin, colloidal albumin, zein, casein, a cellulose derivative, a synthetic hydrophilic colloid such as polyvinyl alcohol or poly-N-vinyl pyrrolidone. If desired, compatible mixtures of two or more of these colloids can be employed for dispersing the silver halide.

The light-sensitive silver halide emulsions used in the preparation of a colour photographic element according to the present invention can be sensitized chemically as well as spectrally. They can be sensitized chemically by carrying out the ripening in the presence of small amounts of sulphur-containing compounds such as allyl thiocyanate, allyl thiourea, or sodium thiosulphate. The emulsions can also be sensitized by means of reducing agents e.g. tin compounds as described in FR-A 1,146,955 and in BE-A 568,687, imino-aminomethane sulphinic acid compounds as described in GB-A 789,823 and small amounts of noble metal compounds such as gold, platinum, palladium, iridium, ruthenium, and rhodium compounds. They can be sensitized spectrally by means of cyanine and merocyanine dyes.

The silver halide emulsions can also comprise compounds that sensitize the emulsions by development acceleration e.g. compounds of the polyoxyalkylene type such as alkylene oxide condensation products as described i.a. in US-A 2,531,831 - 2,533,990 - 4,038,075- 4,292,400, in GB-A 920,637 - 940,051 - 945,340 - 991,608 and 1,091,705, and onium derivatives of amino-N-oxides as described in GB-A 1,121,696.

Further, the emulsions may comprise stabilizers e.g. heterocyclic nitrogen-containing thioxo compounds such as benzothiazoline-2-thione and 1-phenyl-2-tetrazoline-5-thione and compounds of the hydroxytriazolo-pyrimidine type e.g. those described in EP-A 0,190,375. They can also be stabilized with mercury compounds such as the mercury compounds described in BE-A 524,121 and 677,337, and in GB-A 1,173,609.

The light-sensitive emulsions containing the alkali-labile dye-forming couplers used in accordance with the invention may also comprise any other kind of ingredient such as those described for such emulsions in Research Disclosure N° 17,643 of December 1978, in particular development-inhibitor-releasing compounds and competing couplers. Such compounds and couplers can be incorporated in layers in water-permeable relationship with the emulsion layers containing the dye-forming couplers used in accordance with the present invention.

Normally, the alkali-labile non-diffusing cyan-forming couplers for use in accordance with the present invention are incorporated into (a) red-sensitized silver halide emulsion layer(s) of a colour photographic multilayer element, the alkali-labile non-diffusing magenta-forming couplers for use in accordance with the present invention being normally incorporated into (a) green-sensitized silver halide emulsion layer(s) and the alkali-labile non-diffusing yellow-forming couplers for use in accordance with the present invention being normally incorporated into (a) blue-sensitive silver halide emulsion layer(s). Such colour photographic multilayer element preferably comprises a support, (a) red-sensitized silver halide emulsion layer(s) with (a) cyan-forming coupler(s), (a) green-sensitized silver halide emulsion layer(s) with (a) magenta-forming coupler(s), and (a) blue-sensitive silver halide emulsion layer(s) with (a) yellow-forming coupler(s), at least one of these couplers being an alkali-labile dye-forming coupler corresponding to one of the general

formulae I to V.

The emulsions can be coated on a wide variety of photographic supports. Typical supports include cellulose ester film, polyvinylacetal film, polystyrene film, polyethylene terephthalate film and related films or resinous materials, as well as glass and paper e.g. polyethylene- coated paper.

In the production of photographic colour images with the aid of a colour photographic element according to the present invention said element comprising exposed silver halide emulsion layer(s) is developed with aromatic primary amino developing substance(s) in the presence of at least one alkali-labile dye-forming coupler corresponding to one of the general formulae I to V. All colour developing agents capable of forming azomethine dyes can be utilized as developers. Suitable developing agents are aromatic compounds in particular p-phenylene diamines e.g. N,N-diethyl-p-phenylene diamine.

The following examples illustrate the present invention.

## EXAMPLE 1

114.6 g of a spectrally unsensitized silver chloride emulsion comprising per kg an amount of 54 g of gelatin and an amount of silver halide equivalent to 134 g of silver nitrate were diluted with 50 g of a 20 % by volume solution of gelatin in 38 ml of distilled water.

Dispersions of three different cyan-forming couplers as specified in Table 2 hereinafter were made by dissolving a given coupler in an equal amount of dibutyl phthalate and dispersing the resulting solution in an aqueous solution of gelatin by means of an ultrasonic power generator.

TABLE 2: Couplers corresponding to the structural formula:

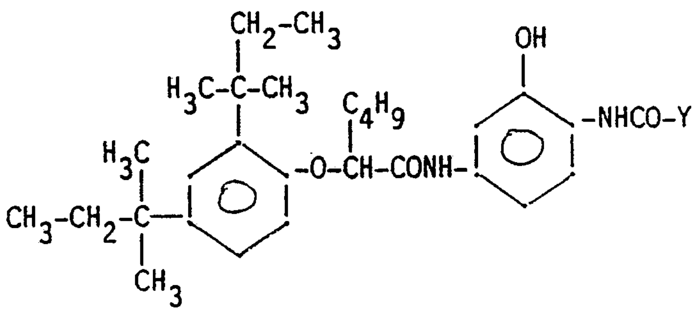

Comparison coupler I (alkali-stable) wherein Y represents 4-cyanoanilino (disclosed in EP-A 0,028,099)
Comparison coupler II (alkali-stable) wherein Y represents ethoxy (prepared as described in Preparation 3 hereinafter)
Coupler 1 according to the invention (alkali-labile)
wherein Y represents phenoxy

## PREPARATION 3: Comparison coupler II (alkali-stable)

An amount of 8.4 g of sodium hydrogen carbonate and 10 ml of ethyl chloroformate is added to a suspension of 22.7 g (0.05 mol) of 5-[2-(2,4-di-tert-pentylphenoxy)-hexanamido]-2-aminophenol in 300 ml of acetonitrile. The resulting mixture is heated for 2 h at 60 °C and poured out in water. The precipitate is dried and then recrystallized from ethanol.
Yield: 42 g (80%). Melting point: 142 °C.

Each of the resulting three dispersions was added to a batch of the same unsensitized silver halide emulsion.

Each of the three emulsion batches was coated on a subbed polyethylene terephthalate film support in a ratio of 2.6 g of gelatin, 2 g of silver chloride calculated as silver nitrate, and 0.5 g of the coupler specified in Table 2 per m2 of dried emulsion layer. The dry emulsion layer of each sample was covered with a gelatin antistress layer in a ratio of 2.6 g of gelatin per m2.

The resulting three materials were identical except for the composition of the coupler.

Each resulting material was cut in two so that samples A1 and A2 having the same composition, both comprising Comparison coupler I, samples B1 and B2 having a same composition, both comprising Comparison coupler II, and samples C1 and C2 having the same composition, both comprising Coupler 1 according to the present invention, were obtained.

The samples A1, B1, and C1 were exposed in a Herrnfeld sensitometer for 1/20th second through a step wedge with a constant of 0.10. The exposed samples A1, B1, and C1 were colour-developed for 2 min in a developing bath at 40° C (pH 10.6) having the following composition:

water 900 ml
anhydrous sodium sulphite 4.35 g
2-amino-5-diethylaminotoluene hydrochloride 2.95 g
anhydrous sodium carbonate 17.1 g
anhydrous sodium bromide 1.72 g
sulphuric acid (7.0 N) 0.62 ml
water to make 1000 ml

The developed samples A1, B1, and C1 were rinsed in water for 2 min, then bleach-fixed for 3 min in a bath (pH 6.30) having the following composition:

water 700 ml
anhydrous sodium sulphite 10 g
ethylenediamine tetraacetic acid tetrasodium salt 13 g
iron ethylenediamine tetraacetic acid monosodium salt 50 g
ammonium bromide 10 g
ethylenediamine tetraacetic acid 5 g
ammonium thiosulphate 150 g
water to make 1000 ml

The bleach-fixed samples A1, B1, and C1 were rinsed in water for 3 min and dried.

By means of a MACBETH (trade name) densitometer RD-919 in the Status A mode the values of maximum density (D max) in the samples A1, B1, and C1 were measured through a red filter.

The samples A2, B2, and C2 were immersed for 2 min in a 1% aqueous solution of sodium hydroxide comprising 5 g of benzotriazole as fog-inhibiting agent and afterwards exposed identically as the samples A1, B1, and C1. Subsequently, the samples A2, B2, and C2 were processed identically as the samples A1, B1, and C1 and the values of D max in samples A2, B2, and C2 were also measured in the same way as described above.

The results obtained for D max in the samples A1, B1, C1 and in the samples A2, B2, C2 respectively are listed in the following Table 3.

TABLE 3

| Samples | D max |
|---|---|
| Sample A1 (Comparison coupler I) | 2.41 |
| Sample A2 (Comparison coupler I) | 2.32 |
| Sample B1 (Comparison coupler II) | 2.30 |
| Sample B2 (Comparison coupler II) | 2.26 |
| Sample C1 (Coupler 1 according to invention) | 2.56 |
| Sample C2 (Coupler 1 according to invention) | 0.58 |

These results show that the alkali-labile coupler 1 according to the invention is unstable in a strong

alkaline medium (Sample C2) and consequently becomes incapable of forming image dye towards the end of the colour development.

EXAMPLE 2

Samples D1 and D2, E1 and E2, and F1 and F2 were made identically as described for the samples A1, A2, B1, B2, C1, and C2 in Example 1. They were exposed for the purpose of measuring the modulation transfer function (MTF).

The samples D1, E1, and F1 were colour-developed for 2 min in a developing bath (40 °C) having the composition described in Example 1 and having a pH-value of 10.6. Afterwards, the developed samples D1, E1, and F1 were rinsed, bleach-fixed, rinsed, and dried as described in Example 1. The modulation transfer function (MTF) of each developed sample D1, E1, and F1 was measured by means of a microdensitometer.

The samples D2, E2, and F2 were colour-developed for 2 min in a developing bath (40 °C) comprising the same ingredients and the same amounts thereof as listed in the developing bath described in Example 1 but additionally comprising a concentrated aqueous sodium hydroxide solution in an amount sufficient to adjust the pH-value of the bath to 13. Afterwards, the developed samples D2, E2, and F2 were rinsed, bleach-fixed, rinsed, and dried in the same way as described in Example 1. The MTF of each developed sample D2, E2, and F2 was also measured.

The MTF curves measured for sample D1 comprising the alkali-stable Comparison coupler I, for sample E1 comprising the alkali-stable Comparison coupler II, and for sample F1 comprising the alkali-labile Coupler 1 according to the present invention, all three developed at pH 10.6, as well as the 3 identical samples D2, E2, and F2, developed, however, at pH 13, are shown in the accompanying Figures 1 to 3. The curves obtained for samples D1, E1, and F1 are represented in the Figures 1 to 3 respectively in full line, whereas the curves obtained for samples D2, E2, and F2 are represented in dash line.

It is seen that the MTF in the case of the alkali-stable comparison couplers I and II does not change substantially when the pH-value of the developing bath has been increased to 13.

The alkali-labile coupler 1 according to the present invention offers a much better MTF at high pH-value of 13 than that at the normal alkaline pH-value of 10.7. This means that when colour development is performed in a higher pH-range, in which the coupler besides forming image dye gradually looses its coupling activity, a manifest improvement in sharpness is obtained. In this way no unsharp dye image can be formed by reaction of coupler with laterally diffusing quinonediimine during the final stage of the colour development.

EXAMPLE 3

114.6 g of a spectrally unsensitized silver chloride emulsion comprising per kg an amount of 54 g of gelatin and an amount of silver halide equivalent to 134 g of silver nitrate were diluted with 50 g of a 20 % by volume solution of gelatin in 38 ml of distilled water.

A dispersion of alkali-labile cyan-forming coupler 4 prepared as described in the above Preparation 2 was made by dissolving the coupler in an equal amount of dibutyl phthalate and dispersing the resulting solution in an aqueous solution of gelatin by means of an ultrasonic power generator.

The resulting dispersion was added to the unsensitized silver halide emulsion.

The emulsion was coated on a subbed polyethylene terephthalate film support in a ratio of 2.6 g of gelatin, 2 g of silver chloride calculated as silver nitrate, and 0.5 g of coupler 4 per m2 of dried emulsion layer. The dry emulsion layer was covered with a gelatin antistress layer in a ratio of 2.6 g of gelatin per m2.

The resulting emulsion material was cut in two so that a sample G1 and a sample G2 having exactly the same composition including that of the coupler were obtained.

The sample G1 was exposed in a Herrnfeld sensitometer for 1/20th second through a step wedge with a constant of 0.10. The exposed sample G1 was colour-developed for 2 min at 40 °C in a developing bath as described in Example 1 hereinbefore.

The developed sample G1 was rinsed in water for 2 min, then bleach-fixed for 3 min in a bleach-fixing bath as described in Example 1 hereinbefore.

The bleach-fixed sample G1 was rinsed in water for 3 min and dried.

By means of a MACBETH (trade name) densitometer RD-919 in the Status A mode the value of maximum density (D max) in sample G1 was measured through a red filter.

Sample G2 was exposed identically as sample G1, but, prior to the processing, sample G2 was immersed for 2 min in a 2% aqueous solution of sodium hydroxide comprising per litre 5 g of benzotriazole as fog-inhibiting agent, 25 ml of cyclohexanedimethanol, 15 g of 2-methyl-2-propyl-1,3-propanediol, and 50 ml of dimethylformamide solvent. Subsequently, sample G2 was processed identically as sample G1 and the value of D max in sample G2 was also measured in the same way as described above.

The results obtained for D max in the samples G1 and G2 are listed in the following Table 4.

TABLE 4

| Coupler | D max in Sample G1 | D max in Sample G2 |
|---|---|---|
| Coupler 4 according to the invention | 1.95 | 0.41 |

These results show that the alkali-labile coupler 4 according to the invention is unstable in a strong alkaline medium (Sample G2) and consequently becomes incapable of forming image dye towards the end of the colour development.

EXAMPLE 4

Samples H1, H2, H3, and H4 of emulsion material having exactly the same composition as described in Example 3 were made and exposed for the purpose of measuring the modulation transfer function.

The samples H1 to H4 were colour-developed for 2 min at 40 °C in a developing bath as described in Example 1 hereinbefore, but with different values of alkalinity. The pH-value of the colour-developing baths was adjusted to 10.6 (for sample H1), 11.1 (for sample H2), 11.8 (for sample H3), and 13.3 (for sample H4) respectively, by addition of 1N aqeuous sodium hydroxide solution

The developed samples were rinsed in water for 2 min, then bleach-fixed for 3 min in a bleach-fixing bath as described in Example 1 hereinbefore.

The bleach-fixed samples were rinsed in water for 3 min and dried.

The MTF curve of each developed sample was measured by means of a microdensitometer. The curves obtained for samples H1 to H4 are shown in the accompanying Figure 4. The curve of H1 is represented as a full line, H2 as a dash line, H3 as a dot and dash line, and H4 as a dotted line.

Comparison of the curves in Figure 4 learns that the modulation transfer function improves with rising pH-value of the colour-developing bath.

The alkali-labile coupler 4 according to the present invention offers a much better MTF-value at pH-values 11.8 and 13.3 than at 10.6 and 11.1, thus demonstrating that when colour development is performed in a higher pH-range, in which the coupler besides forming image dye gradually looses its coupling activity, a striking improvement in sharpness is obtained. In this way no unsharp dye image can be formed by reaction of coupler with laterally diffusing quinonediimine during the final stage of the colour development.

**Claims**

1. Colour photographic element comprising a support and coated thereon at least one alkali-permeable light-sensitive silver halide emulsion layer incorporating in said emulsion layer or in an adjacent layer in water-permeable relationship therewith at least one non-diffusing colour coupler, said at least one non-diffusing colour coupler being capable of reacting in alkaline medium with the oxidation product of a p-phenylene diamine colour developing agent to form a cyan, magenta, or yellow dye, characterized in that said non-diffusing colour coupler is an alkali-labile coupler, whose capability to enter into a coupling reaction with oxidized p-phenylene diamine colour developing agent in said alkaline medium is gradually lost as a result of an alkali-induced reaction of said coupler.

2. A colour photographic element according to claim 1, characterized in that said alkali-labile coupler is chosen from the group consisting of cyan-forming phenol and naphthol couplers, magenta-forming 2-pyrazolin-5-one couplers, and yellow-forming acylacetamide couplers.

3. A colour photographic element according to claim 2, characterized in that said alkali-labile coupler is chosen from the group consisting of
- alkali-labile cyan-forming phenol and naphthol couplers carrying at the 2-position a $-CH(CH_3)-SO_2-R$ substituent, wherein R represents an aryl group or a substituted aryl group,
- alkali-labile cyan-forming phenol and naphthol couplers carrying at the 2-position a -NHCO-A-X substituent, wherein A is a polyvalent hetero-atom and X is an aromatic ring system or a heterocyclic ring system,
- alkali-labile magenta-forming 2-pyrazolin-5-one couplers, which on the 1-position carry a phenyl nucleus having in ortho-position a -CO-A-X substituent, wherein A and X have a significance as defined above,
- alkali-labile yellow-forming benzoylacetamide couplers, which in ortho-position on the benzene ring of the benzoyl group carry a -NHCO-A-X substituent, wherein A and X have a significance as defined above, and
- alkali-labile yellow-forming acylacetanilide couplers, which in ortho-position on the benzene ring of the anilide part carry a -NHCO-A-X substituent, wherein A and X have a significance as defined above,

wherein all of said couplers may carry further substituent(s) as customarily used in the art of coupler chemistry.

4. A colour photographic element according to claim 3, characterized in that said alkali-labile coupler is one of the following classes:
- alkali-labile cyan-forming couplers corresponding to the general formula I:

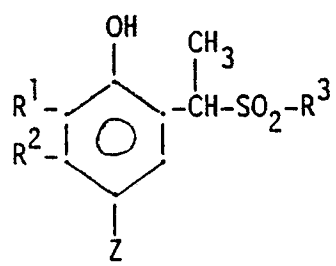

(I)

wherein:

each of $R^1$ and $R^2$, which may be the same or different, is a substituent chosen from the group consisting of hydrogen, a halogen atom, a $C_1$-$C_{18}$ alkyl group, a substituted $C_1$-$C_{18}$ alkyl group, a $C_1$-$C_{18}$ alkoxy group, a substituted $C_1$-$C_{18}$alkoxy group, an acylamido group, a substituted acylamido group, an ureido group, and a substituted ureido group, or $R^1$ and $R^2$ together represent the atoms necessary to complete a fused on carbocyclic ring,

$R^3$ represents an aryl group or a substituted aryl group,

Z is hydrogen in the case of a 4-equivalent coupler or a so-called coupling off group, which splits off upon colour development, thus conferring to the colour coupler a 2-equivalent character,

- alkali-labile cyan-forming couplers corresponding to the general formula II:

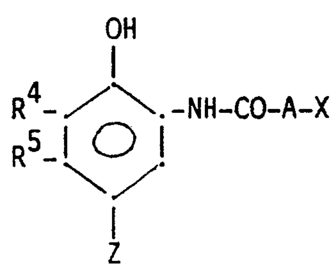

(II)

wherein:

Z has the significance as defined for Z in general formula I,

$R^4$ is a substituent chosen from the group consisting of hydrogen, chloro, an alkyl group, a substituted alkyl group, an alkenyl group, and a substituted alkenyl group,

$R^5$ is a substituent chosen from the group consisting of a $C_5$-$C_{18}$ alkyl group, a substituted $C_5$-$C_{18}$ alkyl group, and a $R^6$-CONH- group, $R^6$ standing for a $C_5$-$C_{18}$ alkyl group or a substituted $C_5$-$C_{18}$ alkyl

group, or
$R^4$ and $R^5$ together represent the atoms necessary to complete a heterocyclic nucleus,

A is a polyvalent hetero-atom,

X is an aromatic ring system or a heterocyclic ring system,

wherein the moiety -A-X is cleavable or releasable gradually from the coupler molecule during prolonged stay in an alkaline medium,

- alkali-labile magenta-forming couplers corresponding to the general formula III:

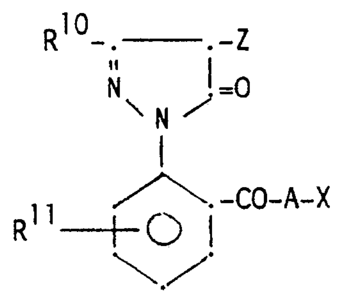

(III)

wherein:

Z has the significance as defined for Z in general formula I,

$R^{10}$ represents a substituent chosen from the group consisting of an alkyl group, a substituted alkyl group, an alkenyl group, a substituted alkenyl group, an aryl group, a substituted aryl group, an alkoxycarbonyl group, a substituted alkoxycarbonyl group, an aryloxycarbonyl group, a substituted aryloxycarbonyl group, an alkoxy group, a substituted alkoxy group, an aryloxy group, an substituted aryloxy group, an alkylthio group, a substituted alkylthio group, an arylthio group, a substituted arylthio group, amino, a substituted amino group, a cycloalkylamino group, a substituted cycloalkylamino group, amido, a substituted amido group, anilino, a substituted anilino group, a N-alkylacylamido group, a substituted N-alkylacylamido group, a N-arylacylamido group, a substituted N-arylacylamido group, a N-alkylureido group, a substituted N-alkylureido group, a N-arylureido group, a substituted N-arylureido group, a N-alkylthioureido group, a substituted N-alkylthioureido group, a carbamoyl group, a substituted carbamoyl group, cyano, a guanidino group, a substituted guanidino group, a heterocyclyl group, a substituted heterocyclyl group, an alkyl-substituted sulphonylamido group, and an aryl-substituted sulphonylamido group,

$R^{11}$ represents a substituent chosen from the group consisting of hydrogen, a halogen atom, an alkyl group, a substituted alkyl group, an alkoxy group, a substituted alkoxy group, cyano, an acylamido group, a substituted acylamido group, an alkyl-substituted carbamoyl group, an aryl-substituted carbamoyl group, an alkylsulphonyl group, a substituted alkylsulphonyl group, an arylsulphonyl group, a substituted arylsulphonyl group, an alkylthio group, a substituted alkylthio group, an arylthio group, and a substituted arylthio group,

A has the significance as defined above,

X has the significance as defined above,

wherein the moiety -A-X is cleavable or releasable gradually from the coupler molecule during prolonged stay in an alkaline medium,

- alkali-labile yellow-forming couplers corresponding to the general formula IV:

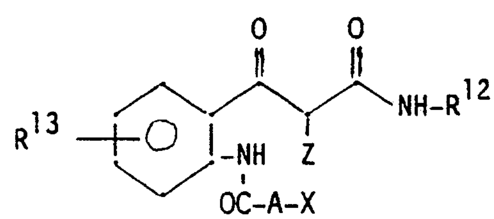

(IV)

wherein:

Z has the significance as defined for Z in general formula I,

$R^{12}$ represents phenyl or phenyl substituted by 1 to 3 substituents, which may be the same or different and are selected from the group consisting of a branched or unbranched $C_1$-$C_{18}$ alkyl group, the alkyl group being substituted or not, an $C_1$-$C_{18}$ alkoxy group, a substituted $C_1$-$C_{18}$ alkoxy group, a halogen atom,

an acylamido group, a substituted acylamido group, a carbamoyl group, a substituted carbamoyl group, a sulphamoyl group, a substituted sulphamoyl group, carboxy, nitro, cyano, an alkylsulphonyl group, a substituted alkylsulphonyl group, an arylsulphonyl group, a substituted arylsulphonyl group, an alkoxycarbonyl group, a substituted alkoxycarbonyl group, an aryloxycarbonyl group, a substituted aryloxycarbonyl group, an alkylthio group, a substituted alkylthio group, an arylthio group, and a substituted arylthio group,

$R^{13}$ represents a substituent chosen from the group consisting of hydrogen, a branched or unbranched $C_1$-$C_{18}$ alkyl group, the alkyl group being substituted or not, an $C_1$-$C_{18}$ alkoxy group, a substituted $C_1$-$C_{18}$ alkoxy group, a halogen atom, an acylamido group, a substituted acylamido group, a carbamoyl group, a substituted carbamoyl group, a sulphamoyl group, a substituted sulphamoyl group, carboxy, nitro, cyano, an alkylsulphonyl group, a substituted alkylsulphonyl group, an arylsulphonyl group, a substituted arylsulphonyl group, an alkoxycarbonyl group, a substituted alkoxycarbonyl group, an aryloxycarbonyl group, a substituted aryloxycarbonyl group, an alkylthio group, a substituted alkylthio group, an arylthio group, and a substituted arylthio group,

A has the significance as defined above,

X has the significance as defined above,

wherein the moiety -A-X is cleavable or releasable gradually from the coupler molecule during prolonged stay in an alkaline medium,

- alkali-labile yellow-forming couplers corresponding to the general formula V:

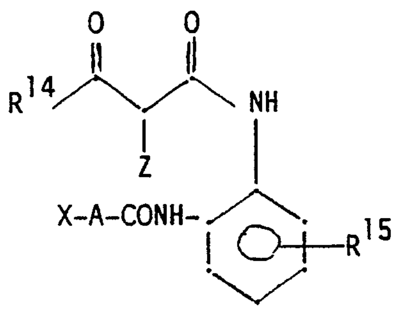

(V)

wherein:

Z has the significance as defined for Z in general formula I,

$R^{14}$ represents a substituent chosen from the group consisting of a W branched or unbranched $C_1$-$C_{18}$ alkyl group, an alkoxyalkyl group, a substituted alkoxyalkyl group, a cycloalkyl group, a substituted cycloalkyl group, a heterocyclyl group, a substituted heterocyclyl group, an aryl group, and an aryl group substituted with a substituent chosen from the group consisting of alkyl, alkoxy, a halogen atom, cyano, an acyl group, a substituted acyl group, an acylamido group, a substituted acylamido group, a carbamoyl group, a substituted carbamoyl group, a sulphamoyl group, a substituted sulphamoyl group, a sulphonamido group, a substituted sulphonamido group, carboxy, an alkylthio group, a substituted alkylthio group, an arylthio group, and a substituted arylthio group,

$R^{15}$ represents a substituent chosen from the group consisting of hydrogen, a branched or unbranched $C_1$-$C_{18}$ alkyl group, the alkyl group being substituted or not, an $C_1$-$C_{18}$ alkoxy group, a substituted $C_1$-$C_{18}$ alkoxy group, a halogen atom, an acylamido group, a substituted acylamido group, a carbamoyl group, a substituted carbamoyl group, a sulphamoyl group, a substituted sulphamoyl group, carboxy, nitro, cyano, an alkylsulphonyl group, a substituted alkylsulphonyl group, an arylsulphonyl group, a substituted arylsulphonyl group, an alkoxycarbonyl group, a substituted alkoxycarbonyl group, an aryloxycarbonyl group, a substituted aryloxycarbonyl group, an alkylthio group, a substituted alkylthio group, an arylthio group, and a substituted arylthio group,

A has the significance as defined above,

X has the significance as defined above,

wherein the moiety -A-X is cleavable or releasable gradually from the coupler molecule during prolonged stay in an alkaline medium.

5. A colour photographic element according to any of claims 1 to 4, characterized in that said element comprises a support, (a) red-sensitized silver halide emulsion layer(s) with (a) cyan-forming coupler(s), (a) green-sensitized silver halide emulsion layer(s) with (a) magenta-forming coupler(s), and (a) blue-sensitive silver halide emulsion layer(s) with (a) yellow-forming coupler(s), at least one of these couplers being a said alkali-labile coupler.

6. A colour photographic element according to claim 5, characterized in that said alkali-labile coupler corresponds to one of the general formulae I to V defined in claim 4.

7. Non-diffusing colour couplers capable of reacting with the oxidation product of a colour developing agent to form a dye, characterized in that the capability of said non-diffusing colour coupler to enter into a coupling reaction with oxidized colour developing agent gradually extinguishes in alkaline medium.

8. A non-diffusing colour coupler according to claim 7, characterized in that said non-diffusing coupler is selected from the classes of couplers defined in claim 4.

9. Method of avoiding lack of definition in dye images produced by chromogenic development including a coupling reaction in alkaline medium between oxidized p-phenylene diamine developing agent and at least one non-diffusing colour coupler, said lack of definition being caused by the coupling reaction taking place also undesiredly between said coupler and any part of said oxidized p-phenylene diamine developing agent that towards the end of said chromogenic development laterally diffuses at the edges of the image areas, characterized in that towards the end of said chromogenic development said at least one coupler is gradually rendered incapable of taking part in said coupling reaction as a consequence of an alkali-induced inactivation reaction of said coupler, said inactivation reaction being controlled by the alkalinity of said alkaline medium and in proportion to the degree of said alkalinity.

10. A method according to claim 9, characterized in that said coupler is chosen from the group consisting of cyan-forming phenol and naphthol couplers, magenta-forming 2-pyrazolin-5-one couplers, and yellow-forming acylacetamide couplers.

11. A method according to claim 10, characterized in that said coupler is one of the classes defined in claim 3.

12. A method according to claim 10, characterized in that said coupler is one of the classes defined in claim 4.

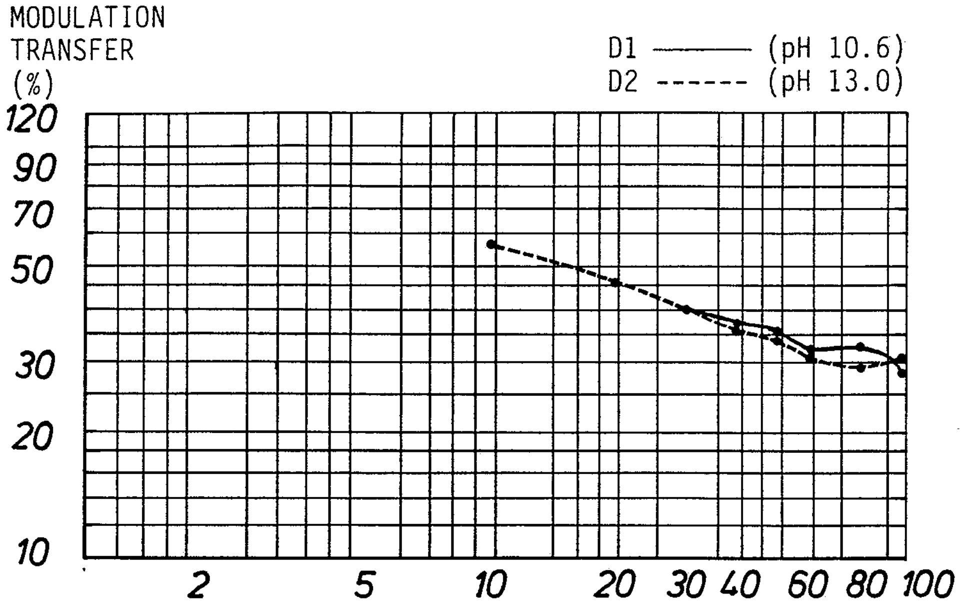
MODULATION
TRANSFER
(%)
D1 ——— (pH 10.6)
D2 ------ (pH 13.0)
120
90
70
50
30
20
10
2
5
10
20
30
40
60
80
100
FREQUENCY
(LINES/MM)

FIG. 1

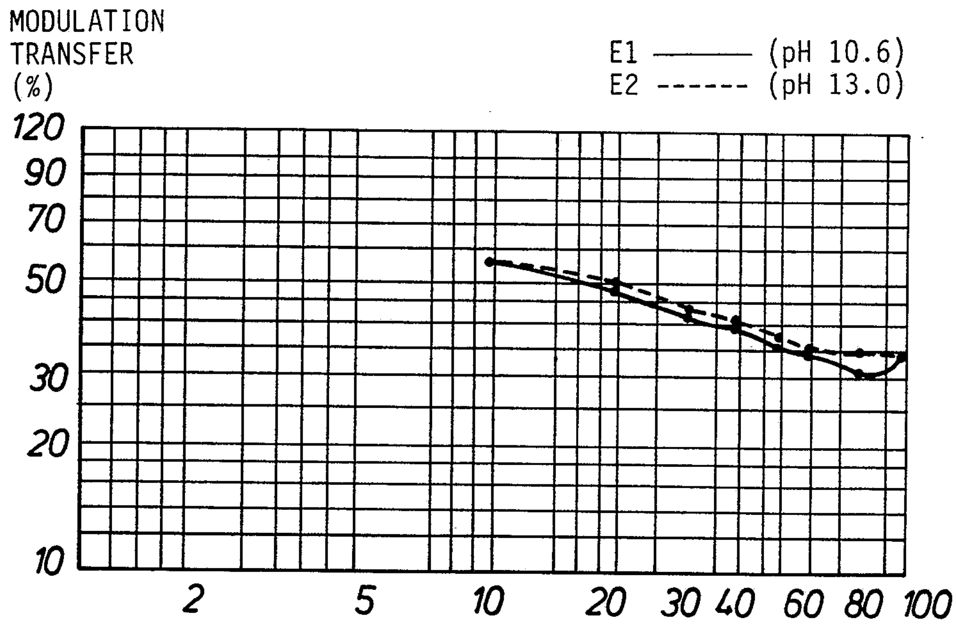
MODULATION
TRANSFER
(%)
E1 ——— (pH 10.6)
E2 ------ (pH 13.0)
120
90
70
50
30
20
10
2
5
10
20
30
40
60
80
100
FREQUENCY
(LINES/MM)

FIG. 2

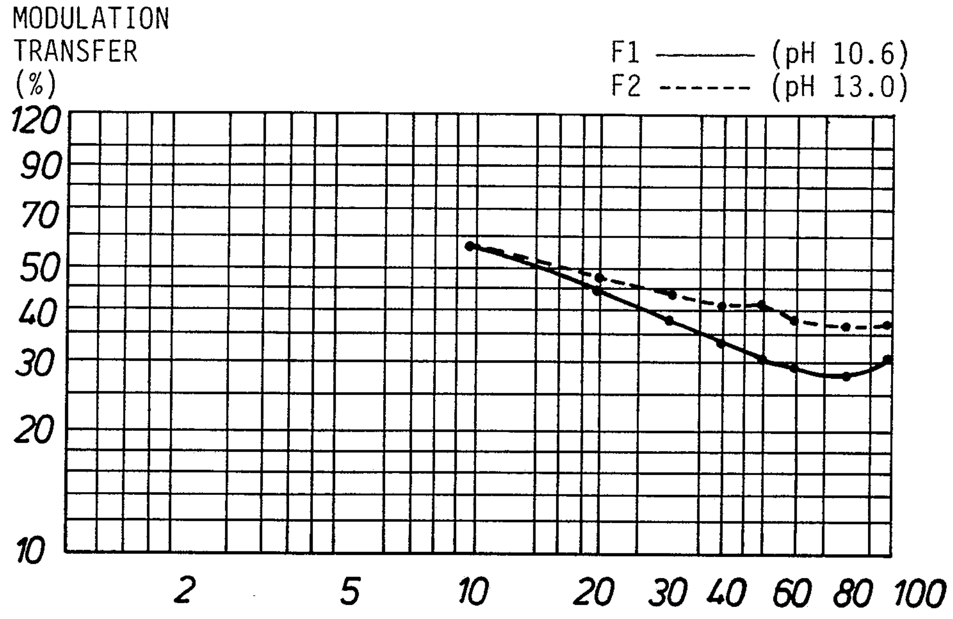
MODULATION
TRANSFER
(%)
F1 (pH 10.6)
F2 (pH 13.0)
120
90
70
50
40
30
20
10
2
5
10
20
30
40
60
80
100
FREQUENCY
(LINES/MM)

FIG. 3

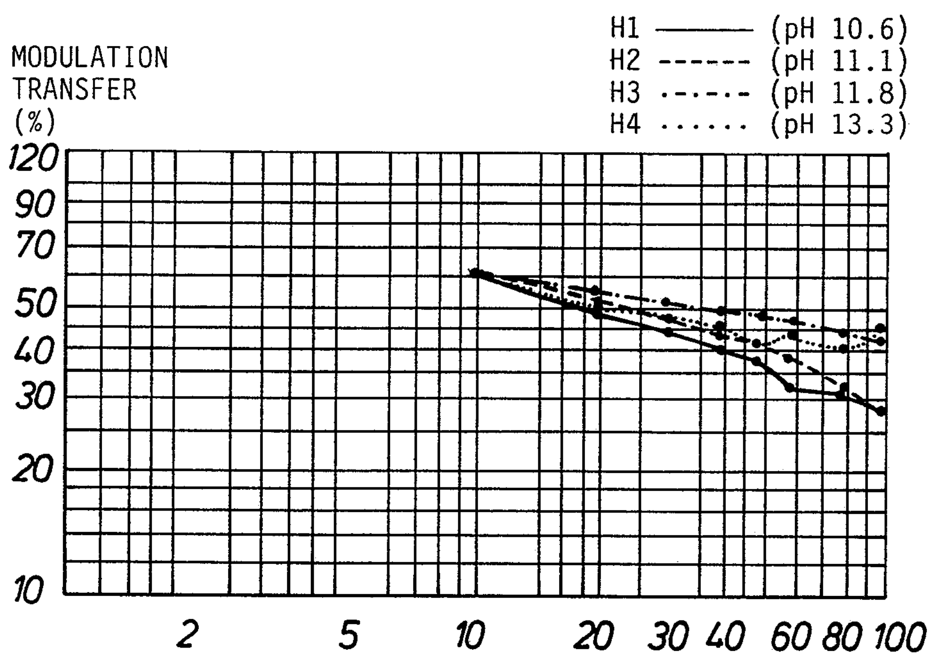
H1 (pH 10.6)
H2 (pH 11.1)
H3 (pH 11.8)
H4 (pH 13.3)
MODULATION
TRANSFER
(%)
120
90
70
50
40
30
20
10
2
5
10
20
30
40
60
80
100
FREQUENCY
(LINES/MM)

FIG. 4

European Patent Office

# EUROPEAN SEARCH REPORT

Application Number

EP 87 20 2040

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | PATENT ABSTRACTS OF JAPAN, vol. 11, no. 299 (P-621)[2746], 29th September 1987; & JP-A-62 91 947 (FUJI PHOTO FILM CO. LTD) 27-04-1987 * Abstract * & US-A-4 725 530 (H. KOBAYASHI et al.) 16-02-1988, claim 1 --- | 1-3 | G 03 C 7/32 |
| A | EP-A-0 173 361 (AGFA-GEVAERT) * Page 6, last paragraph - page 9, last but one paragraph * ----- | | |

TECHNICAL FIELDS SEARCHED (Int. Cl.4)

G 03 C 7

The present search report has been drawn up for all claims

| Place of search | Date of completion of the search | Examiner |
|---|---|---|
| THE HAGUE | 29-06-1988 | PHILOSOPH L.P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)